(19) Europäisches Patentamt
European Patent Office
Office européen des brevets

(11) EP 3 950 918 A1

(12) **EUROPEAN PATENT APPLICATION**
published in accordance with Art. 153(4) EPC

(43) Date of publication:
**09.02.2022 Bulletin 2022/06**

(21) Application number: **20783742.8**

(22) Date of filing: **27.03.2020**

(51) International Patent Classification (IPC):
***C12M 1/00*** (2006.01) ***C12M 3/00*** (2006.01)
***C12N 5/00*** (2006.01) ***C12N 5/07*** (2010.01)

(52) Cooperative Patent Classification (CPC):
**C12M 1/00; C12M 3/00; C12N 5/00**

(86) International application number:
**PCT/JP2020/014013**

(87) International publication number:
**WO 2020/203769 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019 JP 2019068406**

(71) Applicant: **SEKISUI CHEMICAL CO., LTD.**
**Osaka-shi**
**Osaka**
**530-8565 (JP)**

(72) Inventors:
- **HANEDA, Satoshi**
  **Mishima-gun, Osaka 618-0021 (JP)**
- **MANABE, Yuriko**
  **Mishima-gun, Osaka 618-0021 (JP)**
- **ISHII, Ryoma**
  **Mishima-gun, Osaka 618-0021 (JP)**
- **IGUCHI, Hiroki**
  **Mishima-gun, Osaka 618-0021 (JP)**
- **YAMAUCHI, Hiroshi**
  **Mishima-gun, Osaka 618-0021 (JP)**
- **OOMURA, Takahiro**
  **Hasuda-shi, Saitama 349-0198 (JP)**

(74) Representative: **Ter Meer Steinmeister & Partner**
**Patentanwälte mbB**
**Nymphenburger Straße 4**
**80335 München (DE)**

(54) **CELL CULTURE SCAFFOLD MATERIAL, CELL CULTURE VESSEL, CELL CULTURE FIBER AND METHOD FOR CULTURING CELL**

(57) Provided is a scaffolding material for cell culture having suitable hydrophilicity and strength, high fixation of cells after seeding, and highly efficient cell proliferation. The scaffolding material for cell culture according to the present invention has a dispersion component $\gamma^d$ of the surface free energy of 24.5 mJ/m$^2$ or more and less than 45.0 mJ/m$^2$, and a dipole component $\gamma^p$ of the surface free energy of 1.0 mJ/m$^2$ or more and less than 20.0 mJ/m$^2$.

EP 3 950 918 A1

[FIG. 1.]

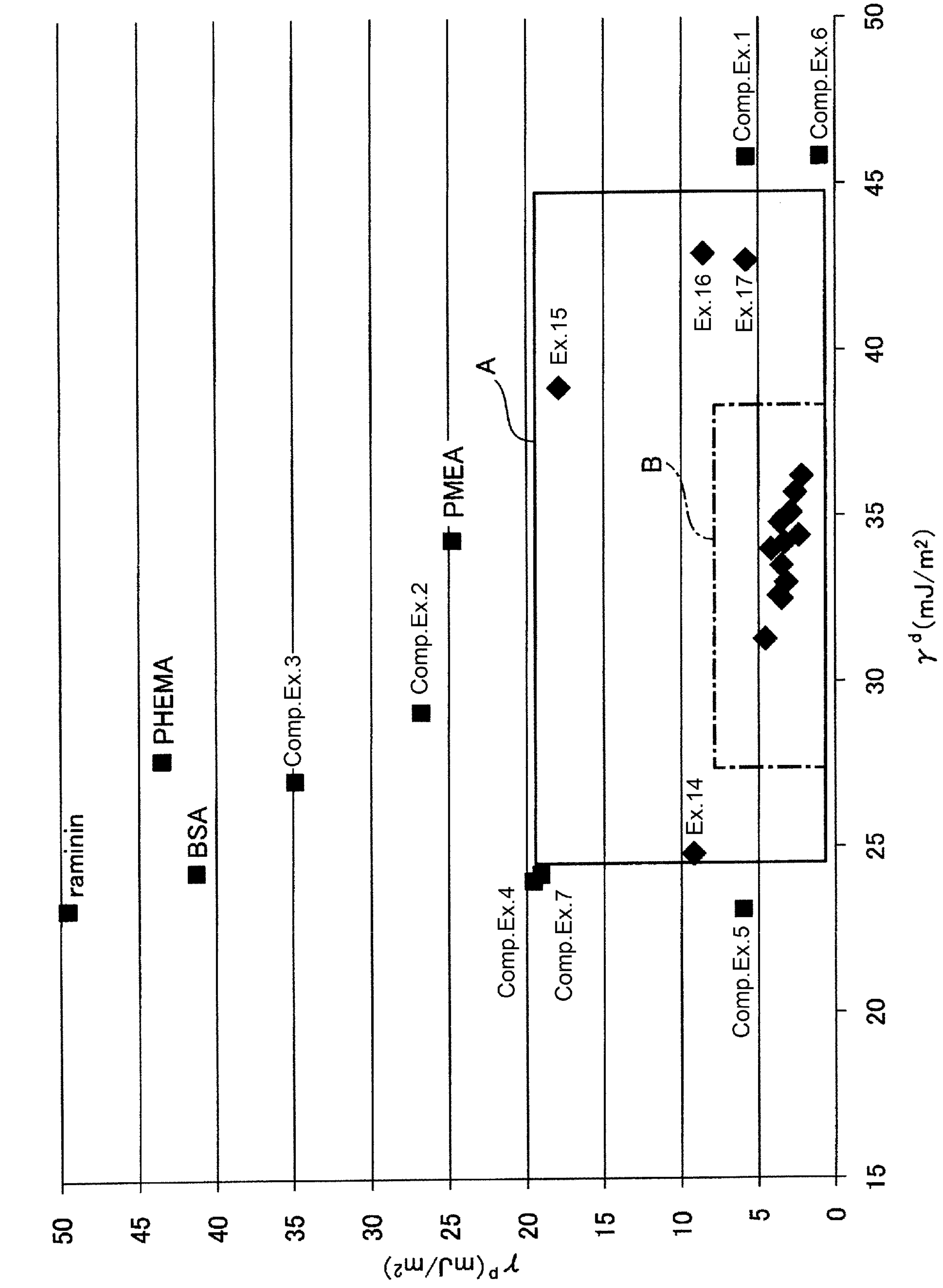
raminin
PHEMA
BSA
Comp.Ex.3
Comp.Ex.2
PMEA
A
Ex.15
B
Ex.14
Ex.16
Ex.17
Comp.Ex.1
Comp.Ex.6
Comp.Ex.4
Comp.Ex.7
Comp.Ex.5
50
45
40
35
30
25
20
15
10
5
0
γp(mJ/m²)
15
20
25
30
35
40
45
50
γd(mJ/m²)

## Description

### TECHNICAL FIELD

The present invention relates to a scaffolding material for cell culture used for culturing a cell. The present invention also relates to a container for cell culture, a fiber for cell culture and a method for culturing a cell obtained by or conducted by using the scaffolding material for cell culture.

### BACKGROUND ART

Animal cells such as human, mouse, rat, pig, cattle and monkey cells are used in research and development in academic fields, drug development fields, regenerative medicine fields and the like. As scaffold materials used for culturing an animal cell, adhesive proteins such as laminin and vitronectin, and natural polymer materials such as Matrigel derived from mouse sarcoma are used.

Furthermore, as shown in Patent Documents 1 to 3 below, scaffold materials obtained by using a synthetic resin are also known.

Patent Document 1 below discloses a carrier for cell culture composed of a molded product made of a polyvinyl acetal compound or a molded product made of the polyvinyl acetal compound and a water-soluble polysaccharide, having a degree of acetalization of the polyvinyl acetal compound of 20 to 60 mol%.

Patent Document 2 below discloses a scaffold material made of hydrogel, for culturing an undifferentiated cell in an undifferentiated state.

Furthermore, Patent Document 3 below discloses a method for culturing a cell by which pluripotent stem cells are maintained in an undifferentiated state, including the step of culturing the pluripotent stem cells on an incubator having a surface coated with a polyrotaxane block copolymer.

### Related Art Documents

### Patent Documents

Patent Document 1: JP 2006-314285 A
Patent Document 2: JP 2010-158180 A
Patent Document 3: JP 2017-23008 A

### SUMMARY OF THE INVENTION

### PROBLEMS TO BE SOLVED BY THE INVENTION

By using natural polymer materials as scaffold materials, the fixation of cells after seeding can be enhanced. However, natural polymer materials are expensive, have large variations between lots because they are naturally occurring substances, and have safety concerns due to animal-derived components.

On the other hand, scaffold materials obtained by using a synthetic resin as described in Patent Documents 1 to 3 are cheaper, have less variation between lots, and are excellent in safety as compared with scaffold materials obtained by using a natural polymer material. However, the scaffold materials obtained by using a synthetic resin as described in Patent Documents 1 to 3 are extremely hydrophilic. Therefore, the scaffold materials are swelled by a liquid medium so that the scaffold materials are peeled off etc. In this way, the strength may be inferior. In addition, in the scaffold materials obtained by using a synthetic resin as described in Patent Documents 1 to 3, the fixation of cells after seeding is so low that the cells do not proliferate sufficiently.

Conventionally, it has been difficult to prepare a scaffolding material for cell culture which can have suitable hydrophilicity and strength, high fixation of cells after seeding, and highly efficient cell proliferation.

An object of the present invention is to provide a scaffolding material for cell culture having suitable hydrophilicity and strength, high fixation of cells after seeding, and highly efficient cell proliferation. Another object of the present invention is to provide a container for cell culture, a fiber for cell culture and a method for culturing a cell obtained by or conducted by using the scaffolding material for cell culture.

## MEANS FOR SOLVING THE PROBLEMS

According to a broad aspect of the present invention, a scaffolding material for cell culture is provided, having a dispersion component $\gamma^d$ of the surface free energy of 24.5 mJ/m$^2$ or more and less than 45.0 mJ/m$^2$, and a dipole component $\gamma^p$ of the surface free energy of 1.0 mJ/m$^2$ or more and less than 20.0 mJ/m$^2$.

In a certain aspect of the scaffolding material for cell culture according to the present invention, the scaffolding material for cell culture includes a synthetic resin.

In another certain aspect of the scaffolding material for cell culture according to the present invention, the synthetic resin has at least one skeleton of a polyvinyl acetal skeleton and a poly(meth)acrylic ester skeleton.

In yet another certain aspect of the scaffolding material for cell culture according to the present invention, the synthetic resin is a polyvinyl acetal resin.

According to a broad aspect of the present invention, a scaffolding material for cell culture containing a synthetic resin is provided, the synthetic resin containing a polyvinyl acetal resin, and the degree of acetalization of the polyvinyl acetal resin being higher than 60 mol%.

In a certain aspect of the scaffolding material for cell culture according to the present invention, the polyvinyl acetal resin has at least one structural unit selected from the group consisting of a structural unit having an amine structure, a structural unit having an imine structure and a structural unit having an amide structure.

In another certain aspect of the scaffolding material for cell culture according to the present invention, the polyvinyl acetal resin has a total content of the structural unit having an amine structure, the structural unit having an imine structure and the structural unit having an amide structure of 0.1 mol% or more and 30 mol% or less.

In yet another certain aspect of the scaffolding material for cell culture according to the present invention, the cell is a somatic cell, a stem cell, a progenitor cell or a differentiated cell.

According to a broad aspect of the present invention, a container for cell culture provided with the scaffolding material for cell culture on at least a part of a cell culture region is provided.

According to a broad aspect of the present invention, a fiber for cell culture containing the scaffolding material for cell culture is provided.

According to a broad aspect of the present invention, a method for culturing a cell including using the scaffolding material for cell culture is provided.

In a certain aspect of the method for culturing a cell according to the present invention, a step of seeding a cell mass on the scaffolding material for cell culture is included.

## EFFECT OF THE INVENTION

According to the present invention, there are provided a scaffolding material for cell culture having suitable hydrophilicity and strength, high fixation of cells after seeding, and highly efficient cell proliferation, and a container for cell culture, a fiber for cell culture and a method for culturing a cell obtained by or conducted by using the scaffolding material for cell culture.

## BRIEF DESCRIPTION OF DRAWINGS

[Fig. 1] Fig. 1 is a view showing the relationship between the dispersion component $\gamma^d$ and the dipole component $\gamma^p$ of the surface free energy of main synthetic resins.

[Fig. 2] Fig. 2 is a partially enlarged view of Fig. 1.

[Fig. 3] Fig. 3 is a partially enlarged view of Fig. 1.

## MODES FOR CARRYING OUT THE INVENTION

Hereinafter, a description is made of the present invention with reference to embodiments, but the present invention is not limited to the following embodiments.

[Scaffolding material for cell culture 1]

The first aspect of a scaffolding material for cell culture according to the present invention is one having a dispersion component $\gamma^d$ of the surface free energy of the scaffolding material for cell culture of 24.5 mJ/m$^2$ or more and less than 45.0 mJ/m$^2$, and a dipole component $\gamma^p$ of the surface free energy of 1.0 mJ/m$^2$ or more and less than 20.0 mJ/m$^2$.

The present inventors have found that the above problems can be solved by controlling the surface free energy

of a scaffolding material for cell culture, and thus have completed the present invention.

The dispersion component $\gamma^d$ and the dipole component $\gamma^p$ of the surface free energy are calculated using the Kaelble-Uy theoretical formula. The Kaelble-Uy theoretical formula is a theoretical formula based on the assumption that the total surface free energy $\gamma$ is the sum of the dispersion component $\gamma^d$ and the dipole component $\gamma^p$, as shown in equation (1).

[Equation 1]

$$\gamma = \gamma^d + \gamma^p \qquad (1)$$

In addition, in the Kaelble-Uy theoretical formula, when the surface free energy of the liquid is represented by $\gamma_l$ (mJ/m$^2$), the surface free energy of the solid is represented by $\gamma_s$ (mJ/m$^2$), and the contact angle is represented by $\theta$(°), the following equation (2) is established.

[Equation 2]

$$\gamma_l\,(1 + \cos\theta) = 2\sqrt{\gamma_s^d \gamma_l^d} + 2\sqrt{\gamma_s^p \gamma_l^p} \qquad (2)$$

Accordingly, using two types of liquids whose surface free energy $\gamma_l$ of liquid is known, the dispersion component $\gamma^d$ and the dipole component $\gamma^p$ of the surface free energy of a scaffolding material for cell culture can be determined by measuring the contact angle $\theta$ with respect to a resin film formed using the scaffolding material for cell culture and solving simultaneous equations for $\gamma_s^d$ and $\gamma_s^p$.

In the present invention, pure water and diiodomethane are used as the two types of liquids whose surface free energy $\gamma_l$ is known.

The contact angle $\theta$ is measured as follows using a contact angle meter (for example, "DMo-701" manufactured by Kyowa Interface Science, Inc.).

Onto the surface of the resin film formed using the scaffolding material for cell culture, 1 $\mu$L of pure water or diiodomethane is dropped. The angle between the pure water 30 seconds after the dropping and the resin film is defined as the contact angle $\theta$ with respect to pure water. Similarly, the angle between the diiodomethane 30 seconds after the dropping and the resin film is defined as the contact angle $\theta$ with respect to diiodomethane.

The scaffolding material for cell culture preferably contains a synthetic resin from the viewpoint of suitably adjusting the dispersion component $\gamma^d$ and the dipole component $\gamma^p$ of the surface free energy. The synthetic resin preferably has at least one skeleton of a polyvinyl acetal skeleton and a poly(meth)acrylic ester skeleton from the viewpoint of more suitably adjusting the dispersion component $\gamma^d$ and the dipole component $\gamma^p$ of the surface free energy.

Fig. 1 is a view showing the relationship between the dispersion component $\gamma^d$ and the dipole component $\gamma^p$ of the surface free energy of main synthetic resins. Each of Figs. 2 and 3 is a partially enlarged view of Fig. 1. In addition, in Figs. 1 to 3, the results of the synthetic resins used in Examples and Comparative Examples described below are also shown.

From the viewpoint of exerting the effect of the present invention, the scaffolding material for cell culture has a dispersion component $\gamma^d$ of the surface free energy of 24.5 mJ/m$^2$ or more and less than 45.0 mJ/m$^2$, and a dipole component $\gamma^p$ thereof of 1.0 mJ/m$^2$ or more and less than 20.0 mJ/m$^2$.

From the viewpoint of more effectively exerting the effect of the present invention, the dispersion component $\gamma^d$ of the surface free energy of the scaffolding material for cell culture is preferably 28.0 mJ/m$^2$ or more, more preferably 32.8 mJ/m$^2$ or more, and preferably 38.0 mJ/m$^2$ or less, more preferably 36.0 mJ/m$^2$ or less.

From the viewpoint of more effectively exerting the effect of the present invention, the dipole component $\gamma^p$ of the surface free energy of the scaffolding material for cell culture is preferably 2.5 mJ/m$^2$ or more, and preferably 10.0 mJ/m$^2$ or less, more preferably 5.0 mJ/m$^2$ or less.

The dispersion component $\gamma^d$ and the dipole component $\gamma^p$ can be controlled, for example, by appropriately changing the skeleton of the synthetic resin described below.

For example, the dispersion component $\gamma^d$ can be increased by increasing the content of a non-polar functional group in the synthetic resin or by introducing a functional group having a cyclic structure, or can be decreased by reducing the content of a butyl group in the synthetic resin, or the like.

For example, the dipole component $\gamma^p$ can be increased by increasing the content of a polar functional group in the synthetic resin or by introducing a functional group containing an ether structure, or can be decreased by increasing the content of a butyl group, which is a non-polar functional group.

(Synthetic resin)

The synthetic resin refers to a resin mainly composed of a polymer (hereinafter, also simply referred to as "polymer") obtained by polymerizing (including polycondensing) a polymerizable monomer (hereinafter, also simply referred to as "monomer"). The polymer may be a homopolymer obtained by polymerizing only one type of monomer, or a copolymer obtained by polymerizing two or more types of monomers.
Examples of the polymer include a polymer composed of one or more polymerizable monomers of saturated or unsaturated hydrocarbons, aromatic hydrocarbons, saturated or unsaturated fatty acids, aromatic carboxylic acids, saturated or unsaturated ketones, aromatic ketones, saturated or unsaturated alcohols, aromatic alcohols, saturated or unsaturated amines, aromatic amines, saturated or unsaturated thiols, aromatic thiols and organosilicon compounds.
Specific examples of the polymer include polyolefin, polyether, polyvinyl alcohol, a polyvinyl acetal resin, polyester, poly(meth)acrylic ester, an epoxy resin, polyamide, polyimide, polyurethane, polycarbonate, cellulose and polypeptide.
From the viewpoint of further enhancing the fixation of cells, the synthetic resin preferably has at least one skeleton of a polyvinyl acetal skeleton and a poly(meth)acrylic ester skeleton, and a polyvinyl acetal resin is preferable.
From the viewpoint of further enhancing the fixation of cells, the polymer is preferably a poly(meth)acrylic ester or a polyvinyl acetal resin, and a polyvinyl acetal resin is more preferable.
The polymers may be used alone or in combination of two or more. When two or more polymers are combined, they may be used as a mixture, or may be used as a polymer in which the skeletons of the two or more polymers are chemically bonded. When two or more polymers are combined as a synthetic resin, it is preferable to combine poly(meth)acrylic ester and polyvinyl acetal.
In the present specification, "(meth)acrylate" refers to at least one selected from the group consisting of (meth)acrylic ester and (meth)acrylic acid. In addition, poly(meth)acrylate is not only polymers obtained by polymerizing a monomer, (meth)acrylic ester or (meth)acrylic acid, but also includes those obtained by copolymerizing a monomer in addition to (meth)acrylic ester or (meth)acrylic acid.
The (meth)acrylic ester is not particularly limited, but includes alkyl (meth)acrylic esters, cyclic alkyl (meth)acrylic esters, aryl (meth)acrylic esters, (meth)acrylamides, polyethylene glycol (meth)acrylates and phosphorylcholine (meth)acrylates.
Examples of the alkyl (meth)acrylic ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-octyl (meth)acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, lauryl (meth)acrylate, stearyl (meth)acrylate and isotetradecyl (meth)acrylate.
These alkyl (meth)acrylic esters are not particularly limited, but may be substituted with various substituents including an alkoxy group having 1 to 3 carbon atoms and a tetrahydrofurfuryl group. Examples of the alkyl (meth)acrylic ester include methoxyethyl acrylate and tetrahydrofurfuryl acrylate.
Examples of the cyclic alkyl (meth)acrylic ester include cyclohexyl (meth)acrylate and isobornyl (meth)acrylate.
Examples of the aryl (meth)acrylic ester include phenyl (meth)acrylate and benzyl (meth)acrylate.
Examples of the acrylamide include (meth)acrylamide, N-isopropyl (meth)acrylamide, N-tert-butyl (meth)acrylamide, N,N'-dimethyl (meth)acrylamide, (3-(meth)acrylamidopropyl) trimethylammonium chloride, 4-(meth)acryloylmorpholine, 3-(meth)acryloyl-2-oxazolidinone, N-[3-(dimethylamino) propyl] (meth)acrylamide, N-(2-hydroxyethyl) (meth)acrylamide, N-methylol (meth)acrylamide and 6-(meth)acrylamidohexanoic acid.
Examples of the polyethylene glycol (meth)acrylate include methoxy-polyethylene glycol (meth)acrylate, ethoxy-polyethylene glycol (meth)acrylate, hydroxy-polyethylene glycol (meth)acrylate, methoxy-diethylene glycol (meth)acrylate, ethoxy-diethylene glycol (meth)acrylate, hydroxy-diethylene glycol (meth)acrylate, methoxytriethylene glycol (meth)acrylate, ethoxy-triethylene glycol (meth)acrylate and hydroxy-triethylene glycol (meth)acrylate.
Examples of the phosphorylcholine (meth)acrylate include 2-(meth)acryloyloxyethyl phosphorylcholine.
Monomers other than the (meth)acrylic esters are not particularly limited, but include (meth)acrylic acids, ethylene and vinyl esters.
The (meth) acrylic esters may be used alone or in combination of two or more.
Note that, in this specification, "(meth)acrylic" means "acrylic" or "methacrylic", and "(meth)acrylate" means "acrylate" or "methacrylate".
From the viewpoint of more effectively exerting fixation of cells, the first aspect of the scaffolding material for cell culture according to the present invention is preferably a combination with the second aspect described below.

[Scaffolding material for cell culture 2]

The second aspect of the scaffolding material for cell culture according to the present invention contains a synthetic resin, the synthetic resin containing a polyvinyl acetal resin, and the degree of acetalization of the polyvinyl

acetal resin being higher than 60 mol%.

The present inventors have found that the above problems can be solved by using a synthetic resin containing a polyvinyl acetal resin having a degree of acetalization of more than 60 mol%, and thus have completed the present invention.

The scaffolding material for cell culture according to the present invention includes an aspect composed of only a polyvinyl acetal resin having a degree of acetalization higher than 60 mol%.

The scaffolding material for cell culture according to the present invention has so suitable hydrophilicity and strength that the fixation of cells after seeding is improved. In particular, in a serum-free medium culture containing no feeder cell or adhesive protein, the initial fixation rate of cells after seeding is improved.

Conventionally, it has not been reported to set the degree of acetalization of a synthetic resin higher than 60 mol% when the synthetic resin is used as a scaffolding material for cell culture. This is because there has been a concern about a decrease in the proportion of hydroxyl groups with an increase in the degree of acetalization, which decreases the hydrophilicity of a scaffolding material for cell culture, leading to a decreased fixation of cells after seeding, and a decrease in permeability of polysaccharides necessary for cell culture and the like. However, the present inventors have found that strength is more important than hydrophilicity, and improving the strength of a scaffolding material for cell culture by setting the degree of acetalization to be higher than 60 mol% allows the fixation of cells after seeding to be improved, and thus have completed the present invention.

Hereinafter, a detailed description is made of the polyvinyl acetal resin.

(Polyvinyl acetal resin)

The polyvinyl acetal resin can be synthesized by acetalizing polyvinyl alcohol with an aldehyde. The polyvinyl acetal resin has an acetyl group, a hydroxyl group and an acetal group on the side chain.

The aldehyde used for acetalizing polyvinyl alcohol is not particularly limited. Examples of the aldehyde include aldehydes having 1 to 10 carbon atoms. The aldehyde may have a chain aliphatic group, a cyclic aliphatic group or an aromatic group. The aldehyde may be a chain aldehyde or a cyclic aldehyde.

The type of the aldehyde includes formaldehyde, acetaldehyde, propionaldehyde, butyraldehyde, pentanal, hexanal, heptanal, octanal, nonanal, decanal, acrolein, benzaldehyde, cinnamaldehyde, perylaldehyde, formylpyridine, formylimidazole, formylpyrrole, formylpiperidine, formyltriazole, formyltetrazole, formylindole, formylisoindole, formylpurine, formylbenzimidazole, formylbenzotriazole, formylquinoline, formylisoquinoline, formylquinoxaline, formylcinnoline, formylpteridine, formylfuran, formyloxolane, formyloxane, formylthiophene, formylthiolane, formylthiane, formyladenine, formylguanine, formylcytosine, formylthymine and formyluracil. The aldehyde may be used alone or in combination of two or more.

The aldehyde is preferably formaldehyde, acetaldehyde, propionaldehyde, butyraldehyde or pentanal, more preferably butyraldehyde. Accordingly, the polyvinyl acetal resin is more preferably a polyvinyl butyral resin.

The average degree of polymerization of the polyvinyl acetal resin is preferably 100 or more, more preferably 200 or more, still more preferably 500 or more, especially preferably 1500 or more, and preferably 6000 or less, more preferably 3000 or less, still more preferably 2500 or less. When the average degree of polymerization is equal to or higher than the above-mentioned lower limit, the strength of the scaffolding material for cell culture can be suitably maintained even when swelled in a liquid medium, so that the cell proliferation is improved. When the average degree of polymerization is equal to or lower than the above-mentioned upper limit, the handleability can be improved, so that the moldability of the scaffolding material for cell culture can be improved.

The degree of acetalization of the polyvinyl acetal resin (degree of butyralization in the case of the polyvinyl butyral resin) is preferably 60 mol% or more, more preferably 65 mol% or more, and preferably 90 mol% or less, more preferably 85 mol% or less. When the degree of acetalization is equal to or higher than the above-mentioned lower limit, the fixation of cells is excellent, and thus cell proliferation can be performed with high efficiency. When the degree of acetalization is equal to or lower than the above-mentioned upper limit, the solubility in solvent can be better.

The polyvinyl acetal resin has a degree of acetylation (an amount of acetyl group) of preferably 0.0001 mol% or more and preferably 5 mol% or less.

The degree of acetal and degree of acetylation of the polyvinyl acetal resin can be measured by $^1$H-NMR (nuclear magnetic resonance spectrum).

The polyvinyl acetal resin may be a resin in which a vinyl compound is copolymerized. The polyvinyl acetal resin may be a copolymer with a vinyl compound. In the present invention, as the polyvinyl acetal resin, a polyvinyl acetal resin obtained by copolymerization with a vinyl group is also considered. The vinyl compound is a compound having a vinyl group ($H_2C=CH$-). The vinyl compound may be a polymer having a constitutional unit having a vinyl group.

The copolymer may be a block copolymer of a polyvinyl acetal resin and a vinyl compound, or a graft copolymer in which a vinyl compound is grafted to a polyvinyl acetal resin. The copolymer is preferably a graft copolymer.

The copolymer can be synthesized, for example, by any of the following methods (1) to (3). (1) A method for

synthesizing a polyvinyl acetal resin including using polyvinyl alcohol in which a vinyl compound is copolymerized. (2) A method for synthesizing a polyvinyl acetal resin including using polyvinyl alcohol and polyvinyl alcohol in which a vinyl compound is copolymerized. (3) A method including graft-copolymerizing a vinyl compound to a pre-graft-copolymerized polyvinyl acetal resin.

The vinyl compound includes ethylene, allylamine, vinylpyrrolidone, maleic anhydride, maleimide, itaconic acid, (meth)acrylic acid, vinylamine and (meth)acrylic ester. The vinyl compound may be used alone or in combination of two or more. Examples of the (meth)acrylic ester include the above-mentioned (meth)acrylic esters.

The graft copolymer contains a graft copolymer having a "unit composed of polyvinyl acetal" and a "unit composed of a vinyl compound" (hereinafter, also simply referred to as "graft copolymer").

In the present invention, the "unit composed of polyvinyl acetal" and the "unit composed of a vinyl compound" refer to a unit composed of "polyvinyl acetal" and "a vinyl compound" present in the graft copolymer.

In addition, a graft copolymer having a unit composed of polyvinyl acetal and a unit composed of a vinyl compound refers to a branched copolymer in which, to a "unit composed of polyvinyl acetal" or a "unit composed of a vinyl compound" composing the main chain, a "unit composed of polyvinyl acetal" or a "unit composed of a vinyl compound" composing a side chain different from the main chain is bonded.

The molecular weight of the graft copolymer is not particularly limited, but it is preferable that the number average molecular weight (Mn) be 10,000 to 600,000, the weight average molecular weight (Mw) be 20,000 to 1,200,000 and the ratio (Mw/Mn) be 2.0 to 40. When the Mn, Mw and Mw/Mn are in such ranges, the strength of the scaffolding material for cell is suitably maintained.

The degree of acetalization in the graft copolymer can be measured by $^{1}H$-NMR in which a soluble component of the graft copolymer in xylene is dissolved in deuterated dimethyl sulfoxide, for example.

From the viewpoint of more effectively exerting the effect of the present invention, the polyvinyl acetal resin preferably has a Bronsted basic group or a Bronsted acidic group, more preferably has a Bronsted basic group. In other words, a part of the polyvinyl acetal resin is preferably modified with a Bronsted basic group or a Bronsted acidic group, more preferably modified with a Bronsted basic group. In this case, in a serum-free medium culture containing no feeder cell or adhesive protein, the initial fixation rate of cells after seeding is improved, and cell culture is easily performed.

In the present specification, a polyvinyl acetal resin having a Bronsted basic group or a Bronsted acidic group on a part of the polyvinyl acetal resin is sometimes referred to as a modified polyvinyl acetal resin.

The Bronsted basic group is a generic term for a functional group that can receive a hydrogen ion $H^+$ from another substance. Examples of the Bronsted basic group include amine-based basic groups such as a substituent having an amine structure, a substituent having an imine structure, a substituent having an amide structure and a substituent having an imide structure.

The polyvinyl acetal resin preferably has at least one structural unit selected from the group consisting of a structural unit having an amine structure, a structural unit having an imine structure, a structural unit having an amide structure and a structural unit having an imide structure. The polyvinyl acetal resin more preferably has at least one structural unit selected from the group consisting of a structural unit having an amine structure, a structural unit having an imine structure and a structural unit having an amide structure.

In the polyvinyl acetal resin, the total content of the structural unit having an amine structure, the structural unit having an imine structure, the structural unit having an amide structure, and the structural unit having an imide structure is preferably 0.1 mol% or more, more preferably 1 mol% or more, and preferably 30 mol% or less, more preferably 10 mol% or less. When the total content is equal to or more than the above-mentioned lower limit and equal to or less than the above-mentioned upper limit, the cell adhesion immediately after seeding can be further enhanced.

In the polyvinyl acetal resin, the total content of the structural unit having an amine structure, the structural unit having an imine structure, and the structural unit having an amide structure is preferably 0.1 mol% or more, more preferably 1 mol% or more, and preferably 30 mol% or less, more preferably 10 mol% or less. When the total content is equal to or more than the above-mentioned lower limit and equal to or less than the above-mentioned upper limit, the cell adhesion immediately after seeding can be further enhanced.

In the present invention, the imine structure refers to a structure having a C=N bond. The polyvinyl acetal resin preferably has an imine structure on the side chain. In addition, the imine structure may be directly bonded to a carbon atom constituting the main chain of the polyvinyl acetal resin, or may be bonded to the main chain via a linking group such as an alkylene group. Note that having the imine structure on the side chain includes having the imine structure on the graft chain of the polyvinyl acetal resin. Examples of the structural unit having an imine structure include a structural unit represented by the following formula (11) or (12).

[Chemical 1]

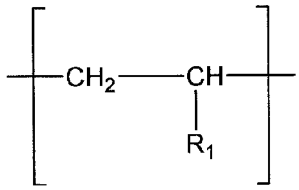

...(11)

In the formula (11), $R_1$ represents a group having an imine structure.

[Chemical 2]

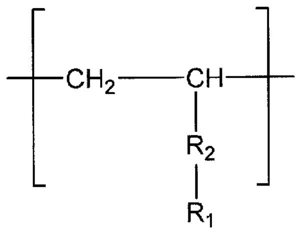

...(12)

In the formula (12), $R_1$ represents a group having an imine structure, and $R_2$ represents an alkylene group. The alkylene group has preferably 1 or more, and preferably 12 or less, more preferably 5 or less carbon atoms. When the carbon number of the alkylene group is equal to or more than the above-mentioned lower limit and equal to or less than the above-mentioned upper limit, the strength of the scaffolding material for cell culture can be enhanced.

The alkylene group includes linear alkylene groups such as a methylene group, ethylene group, trimethylene group, tetramethylene group, pentamethylene group, hexamethylene group, octamethylene group and decamethylene group, branched alkylene groups such as a methyl methylene group, methylethylene group, 1-methylpentylene group and 1,4-dimethylbutylene group, and cyclic alkylene groups such as a cyclopropylene group, cyclobutylene group and cyclohexylene group. The alkylene group is preferably a linear alkyl group such as a methylene group, ethylene group, trimethylene group or tetramethylene group, more preferably a methylene group or ethylene group.

$R_1$ in the formula (11) and $R_1$ in the formula (12) include groups represented by the following formula (13).

[Chemical 3]

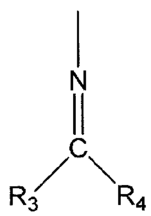

...(13)

In the formula (13), $R_3$ represents a hydrogen atom or a hydrocarbon group having 1 to 18 carbon atoms, and $R_4$ represents a hydrocarbon group having 1 to 18 carbon atoms.

The hydrocarbon group includes a saturated hydrocarbon group, an unsaturated hydrocarbon group and an aromatic hydrocarbon group. The hydrocarbon group may be one composed of only any one of a saturated hydrocarbon group, an unsaturated hydrocarbon group and an aromatic hydrocarbon group, or one in which two or more of them are used.

The saturated hydrocarbon group includes methyl, ethyl, n-propyl, isopropyl, n-butyl, iso-butyl, sec-butyl, tert-butyl, pentyl, hexyl, heptyl, 2-ethylhexyl, octyl, nonyl, decyl, undecyl, dodecyl, tridecyl, tetradecyl, pentadecyl and octadecyl groups. The saturated hydrocarbon group is preferably a methyl group, an ethyl group, an n-propyl group or an n-butyl group.

The aromatic hydrocarbon group includes a phenyl group, toluyl group, xylyl group, t-butylphenyl group and benzyl group.

It is preferable that the structural unit having an imine structure be a structural unit having the structure represented by the formula (11), and in the formula (13), $R_3$ be a hydrogen atom, a methyl group or an ethyl group, and $R_4$ be a methyl group, an ethyl group or a propyl group.

In the polyvinyl acetal resin, the content of the structural unit having an imine structure is preferably 0.1 mol% or more, more preferably 1.0 mol% or more, and preferably 20.0 mol% or less, more preferably 15.0 mol% or less. When the content is equal to or more than the above-mentioned lower limit, the viscosity stability over time can be better. When the content is equal to or less than the above-mentioned upper limit, acetalization can be sufficiently advanced.

In the polyvinyl acetal resin, the ratio of the content of the structural unit having an imine structure to the degree of acetalization (the content of the structural unit having an imine structure/degree of acetalization) is preferably 0.001 or more, preferably 0.5 or less. When the ratio (the content of the structural unit having an imine structure/degree of acetalization) is equal to or more than the above-mentioned lower limit and equal to or less than the above-mentioned upper limit, high strength and excellent adhesiveness can be achieved at the same time, and the durability after adhesion can be improved.

The polyvinyl acetal resin preferably has a structural unit having an imino group (=NH) (a structural unit having an imino structure).

The polyvinyl acetal resin preferably has the imino group on the side chain. In addition, the imino group may be directly bonded to a carbon atom constituting the main chain of the polyvinyl acetal resin, or may be bonded to the main chain via a linking group such as an alkylene group.

The polyvinyl acetal resin preferably has a structural unit having an amine structure or a structural unit having an amide structure.

The polyvinyl acetal resin preferably has the amine structure or the amide structure on the side chain. In addition, the amine structure or the amide structure may be directly bonded to a carbon atom constituting the main chain of the polyvinyl acetal resin, or may be bonded to the main chain via a linking group such as an alkylene group. Furthermore, the amine group in the amine structure may be a primary amine group, a secondary amine group, a tertiary amine group or a quaternary amine group. Among them, a primary amine group is preferable from the viewpoint of enhancing the fixation of cells.

Note that having the amine structure or the amide structure on the side chain includes having the amine structure or the amide structure on the graft chain of the polyvinyl acetal resin.

In particular, the amine structure is preferably $-NH_2$. In the present invention, the amide structure refers to a structure having -C(=O)-NH-. In particular, the structural unit having the amine structure preferably is a structure represented by the following formula (21). In addition, the structural unit having the amide structure preferably has a structure represented by the following formula (31).

[Chemical 4]

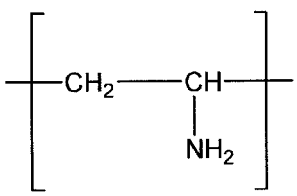

・・・(21)

[Chemical 5]

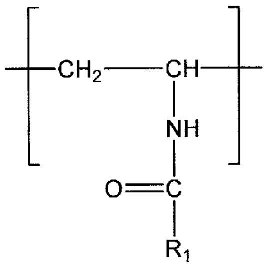

・・・(31)

In the formula (31), $R_1$ represents a hydrogen atom or a hydrocarbon group having 1 to 10 carbon atoms. The hydrocarbon group includes an alkyl group, an alkenyl group, a cycloalkyl group and a cycloalkenyl group.

In the polyvinyl acetal resin, the content of the structural unit having the amine structure or the amide structure is preferably 0.1 mol% or more, more preferably 0.5 mol% or more, and preferably 20 mol% or less, more preferably 10 mol% or less. When the content is equal to or more than the above-mentioned lower limit, additional properties can be improved. When the content is equal to or less than the above-mentioned upper limit, the solubility is not so excessively increased that the modified polyvinyl acetal resin powder can be easily taken out by precipitation method.

In the polyvinyl acetal resin, the total content of the structural unit having the amine structure or the amide structure, and the structural unit having an imine structure is preferably 0.1 mol% or more, more preferably 0.5 mol% or more, and preferably 20 mol% or less, more preferably 10 mol% or less.

In the polyvinyl acetal resin, the ratio of the content of the structural unit having an imine structure with respect to the ratio of the structural unit having an amine structure or an amide structure (the structural unit having an imine structure/the structural unit having an amine structure or an amide structure) is preferably 0.5/99.5 to 99.5/0.5. When the ratio is 0.5/99.5 or more, the viscosity stability over time can be sufficient, whereas when the ratio is 99.5/0.5 or less, the crosslinking performance can be sufficiently exhibited from the viewpoint of improving the fixation of cells. The ratio (the structural unit having an imine structure/the structural unit having an amine structure or an amide structure) is more preferably 5/95 or more and more preferably 90/10 or less.

The content of the structural unit having an imine structure, the content of the structural unit having an imide structure, the content of the structural unit having an amine structure and the content of the structural unit having an amide structure can be measured by $^1$H-NMR (nuclear magnetic resonance spectrum).

The Bronsted acidic group is a generic term for a functional group that can deliver a hydrogen ion $H^+$ to another substance.

The Bronsted acidic group includes a carboxyl group, a sulfonic acid group, a maleic acid group, a sulfinic acid group, a sulfenic acid group, a phosphoric acid group, a phosphonic acid group, and salts thereof. The Bronsted acidic group is preferably a carboxyl group.

The method for modifying the polyvinyl acetal resin with the Bronsted acidic group is not particularly limited, but includes a method for copolymerizing the polyvinyl alcohol with the itaconic acid or (meth)acrylic acid and a method for introducing a Bronsted acidic group into the side chain of the polyvinyl alcohol.

Examples of the method for producing the polyvinyl acetal resin include a method for acetalizing using a conventionally known method a polyvinyl alcohol obtained by saponifying polyvinyl acetate obtained by copolymerizing the monomer having an imine structure with vinyl acetate. In addition, a method may also be used for introducing an imine structure by acetalizing using a conventionally known method a polyvinyl alcohol having a structural unit having an amino group or an amide structure. A method may also be used for acetalizing using a conventionally known method a modified polyvinyl alcohol having an imine structure obtained by post-modifying a polyvinyl alcohol having a structural unit having an amino group or an amide structure. Furthermore, an imine structure may be introduced by post-modifying an unmodified polyvinyl acetal resin. In other words, the modified polyvinyl acetal resin may be an acetalized product of a polyvinyl alcohol having a structural unit having an amino group or an amide structure. Among them, a method is preferable for producing a modified polyvinyl acetal resin having an imine structure by acetalizing a polyvinyl alcohol having a structural unit having an amino group or an amide structure. In particular, when such a method is used, an imine structure can be obtained by adding excessive amounts of aldehyde and acid catalyst for use in acetalization.

In the method for excessively adding aldehyde, it is preferable to add 70 to 150 parts by weight aldehyde to 100 parts by weight a polyvinyl alcohol having a structural unit having an amino group or an amide structure. Particularly, as the aldehyde, acetaldehyde, propionaldehyde, n-butyraldehyde, isobutyraldehyde, n-valeraldehyde and phenylaldehyde are preferable.

In the method for excessively adding an acid catalyst, it is preferable to add the acid catalyst in an amount of 0.5% by weight or more with respect to the whole weight. In addition, it is preferable to add 5.0 to 70.0 parts by weight acid catalyst to 100 parts by weight a polyvinyl alcohol having a structural unit having an amino group or an amide structure. Particularly, as the acid catalyst, hydrochloric acid, nitric acid, sulfuric acid and para-toluenesulfonic acid are preferable. In the case where such a method is used, examples of the method for confirming a structural unit having an amino group or an amide structure, or a structural unit having an imine structure include a confirming method by $^1$H-NMR.

The acetalization can be performed using a known method, and is preferably performed in an aqueous solvent, a mixed solvent of water and an organic solvent having compatibility with water, or an organic solvent. As the organic solvent compatible with water, for example, an alcohol-based organic solvent can be used. Examples of the organic solvent include alcohol-based organic solvents, aromatic organic solvents, aliphatic ester-based solvents, ketone-based solvents, lower paraffin-based solvents, ether-based solvents, amide-based solvents and amine-based solvents.

Examples of the alcohol-based organic solvent include methanol, ethanol, n-propanol, isopropanol, n-butanol and tert-butanol.

Examples of the aromatic organic solvent include xylene, toluene, ethylbenzene and methyl benzoate.

Examples of the aliphatic ester-based solvent include methyl acetate, ethyl acetate, butyl acetate, methyl propionate, ethyl propionate, methyl butyrate, ethyl butyrate, methyl acetoacetate and ethyl acetoacetate.

Examples of the ketone-based solvent include acetone, methyl ethyl ketone, methyl isobutyl ketone, cyclohexanone, methylcyclohexanone, benzophenone and acetophenone.
The lower paraffin-based solvents include hexane, pentane, octane, cyclohexane and decane.
The ether-based solvents include diethyl ether, tetrahydrofuran, ethylene glycol dimethyl ether, ethylene glycol diethyl ether and propylene glycol diethyl ether.
The amide-based solvents include N,N-dimethylformamide, N,N-dimethylacetamide, N-methylpyrrolidone and acetanilide.
The amine-based solvents include ammonia, trimethylamine, triethylamine, n-butylamine, di-n-butylamine, tri-n-butylamine, aniline, N-methylaniline, N,N-dimethylaniline and pyridine.
These organic solvents can be used alone or as a mixture of two or more solvents. Among them, ethanol, n-propanol, isopropanol and tetrahydrofuran are particularly preferable from the viewpoints of solubility in a resin and simplicity during purification.
The acetalization is preferably performed in the presence of an acid catalyst. The acid catalyst is not particularly limited, but includes mineral acids such as sulfuric acid, hydrochloric acid, nitric acid and phosphoric acid, carboxylic acids such as formic acid, acetic acid and propionic acid, and sulfonic acids such as methanesulfonic acid, ethanesulfonic acid, benzenesulfonic acid and para-toluenesulfonic acid. These acid catalysts may be used alone or in combination of two or more compounds. Among them, hydrochloric acid, nitric acid and sulfuric acid are preferable, and hydrochloric acid is particularly preferable.

(Other details of scaffolding material for cell culture)

The scaffolding material for cell culture according to the present invention is used for culturing cells. The scaffolding material for cell culture according to the present invention is used as a scaffold for cells when the cells are cultured. In the scaffolding material for cell culture according to the present invention, it is particularly preferable that a cell mass be seeded. However, in the scaffolding material for cell culture according to the present invention, a cell mass may not be seeded.
The cells include animal cells such as human, mouse, rat, pig, bovine and monkey cells. The cells also include somatic cells, stem cells, progenitor cells and differentiated cells. The somatic cell may also be cancer cells.
The differentiated cells include nerve cells, cardiomyocytes, retinal cells and hepatocytes.
The stem cells include pluripotent stem cells, tissue stem cells and tissue progenitor cells.
The tissue stem cells and tissue progenitor cells refer to cells having an ability to self-renew, belonging to any of the ectodermal, endodermal, mesodermal and germline tissues, and exhibiting a limited ability to differentiate into the constituent cell types of an organ to which they belong.
Examples of the tissue stem cells and tissue progenitor cells include neural stem cells, neural crest stem cells, retinal stem cells, corneal stem cells, keratinocyte epidermal stem cells, melanocyte stem cells, mammary gland stem cells, liver stem cells, intestinal stem cells, respiratory tract stem cells, hematopoietic stem cells, mesenchymal stem cells, cardiac stem cells, vascular endothelial progenitor cells, vascular pericytes, skeletal muscle stem cells, adipose stem cells, renal progenitor cells and sperm stem cells.
The cells are preferably somatic cells, stem cells, progenitor cells or differentiated cells. The somatic cells are preferably cancer cells, nerve cells, cardiomyocytes, retinal cells or hepatocytes.

[Cell culture method]

Various cells can be cultured using the scaffolding material for cell culture according to the present invention. The cells include the above described cells. The scaffolding material for cell culture according to the present invention is hardly swelled with the water in a culture medium, and thus can maintain so suitable hydrophilicity and strength that the fixation rate of cells after seeding can be improved.
The cell culture method preferably includes a step of seeding a cell mass on the scaffolding material for cell culture. The cell mass can be obtained by adding a cell detaching agent to a confluent culture container and uniformly performing crushing by pipetting. The cell detaching agent is not particularly limited, but is preferably an ethylenediamine/phosphate buffer solution. The size of the cell mass is preferably 50 $\mu$m to 200 $\mu$m.
In cell culture, the scaffolding material for cell culture can be used not only for planar culture (two-dimensional culture method) but also for culturing cells on a base material in a state closer to an in-vivo state, such as a porous membrane or a hydrogel (three-dimensional culture method). This is because cells can be efficiently proliferated by using the scaffolding material for cell culture in a bioreactor or the like.
The scaffolding material for cell culture is preferably used in a two-dimensional culture method because it has suitable hydrophilicity and strength.
The container for planar culture (two-dimensional culture method) is not particularly limited for shape and size,

but includes a test plate for cell culture having one or more wells (holes) and a flask for cell culture. The number of wells in the microplate is not limited, but includes, for example, 2, 4, 6, 12, 24, 48, 96 and 384. The shape of the well is not particularly limited, but includes, for example, a perfect circle, ellipse, triangle, square, rectangle, and pentagon. The shape of the bottom surface of the well is not particularly limited, but includes a flat bottom, a round bottom and irregularities.

The material of the test plate for cell culture having one or more wells (holes) or the material of the flask for cell culture are not particularly limited, but includes a polymer resin, metal and inorganic material. The polymer resin includes polystyrene, polyethylene, polypropylene, polycarbonate, polyester, polyisoprene, cycloolefin polymer, polyimide, polyamide, polyamideimide, (meth)acrylic resin, epoxy resin and silicone. The metal includes stainless steel, copper, iron, nickel, aluminum, titanium, gold, silver and platinum. The inorganic material includes silicon oxide (glass), aluminum oxide, titanium oxide, zirconium oxide, iron oxide and silicon nitride.

In addition to the above, the scaffolding material for cell culture can be used in a suspension culture method in which cells are freely suspended and grown in a medium.

[Other embodiments]

In addition to the scaffolding material for cell culture, the present invention provides an invention using the scaffolding material for cell culture as another embodiment.

For example, in the present invention, a carrier (medium) for cell culture containing the scaffolding material for cell culture and a polysaccharide is provided. Various polysaccharides can be used as the polysaccharide without any particular limitation. Among them, water-soluble polysaccharides are preferable.

In addition, in the present invention, a container for cell culture provided with the scaffolding material for cell culture on at least a part of a cell culture region is provided. In the container for cell culture, the scaffolding material for cell culture is preferably in the form of a film, and is preferably a resin film. The container for cell culture includes a vessel body and the scaffolding material for cell culture (resin film) arranged on the surface of the vessel body. The container is not particularly limited as long as it has a resin film on at least a part of the cell culture region, but various containers can be used. As the vessel, a vessel for planar culture, a bioreactor or the like can be used.

In addition, the present invention provides a fiber for cell culture containing the scaffolding material for cell culture. In this case, it is preferable that the scaffolding material for cell culture be applied on the fiber. In addition, the scaffolding material for cell culture may be in a form impregnated or kneaded in the fiber. The fiber for cell culture is suitable for a three-dimensional culture method for cells that are difficult to adhere to a planar structure such as a flask, but easily adhere to a three-dimensional structure such as a fibril-like structure.

The scaffolding material for cell culture may be cross-linked. This is because crosslinking can suppress water swelling and suitably increase the strength. Using a crosslinking agent can provide the crosslinked scaffolding material for cell culture.

The crosslinking agent is not particularly limited, but includes polyalcohol, polycarboxylic acid, hydroxycarboxylic acid, metal soap and polysaccharides.

The polyalcohol is not particularly limited, but includes ethylene glycol, propylene glycol, butanediol, pentanediol, hexanediol, heptanediol, octanediol, nonanediol, decanediol, dodecanediol, undecanediol, diethylene glycol, triethylene glycol, tetraethylene glycol, polyethylene glycol, catechol, pyrogallol, diboronic acid, methylenediboronic acid, ethylenediboronic acid, propylene diboronic acid, phenylenediboronic acid, biphenyldiboronic acid and bisphenol derivatives.

The polycarboxylic acid is not particularly limited, but includes oxalic acid, malonic acid, succinic acid, glutaric acid, adipic acid, pimelic acid, suberic acid, azelaic acid, sebacic acid, phthalic acid and poly(meth)acrylic acid.

The hydroxycarboxylic acid is not particularly limited, but includes glycolic acid, lactic acid, tartronic acid, glyceric acid, hydroxybutyric acid, malic acid, tartaric acid, cytomaric acid, citric acid, isocitric acid, leucic acid, mevalonic acid, pantoic acid, ricinoleic acid, ricineraidic acid, cerebronic acid, quinic acid, shikimic acid, hydroxybenzoic acid, salicylic acid, creosoteic acid, vanillic acid, syringic acid, pyrocatechuic acid, resorcylic acid, protocatechuic acid, gentisic acid, orsellinic acid, gallic acid, mandelic acid, benzilic acid, atrolactic acid, melilotic acid, phloretic acid, coumaric acid, umbellic acid, caffeic acid, ferulic acid, sinapinic acid and hydroxystearic acid.

The metal soap is not particularly limited, but includes salts of fatty acids such as stearic acid, lauric acid, ricinoleic acid and octylic acid with metals such as lithium, sodium, magnesium, calcium, barium, zinc and aluminum.

The polysaccharides are not particularly limited, but include pectin, guar gum, xanthan gum, tamarind gum, carrageenan, propylene glycol, carboxymethylcellulose, amylose, amylopectin, glycogen, cellulose, chitin, agarose, carrageenan, heparin, hyaluronic acid, xyloglucan and glucomannanic acid.

## EXAMPLES

Hereinafter, a description is made of the present invention with reference to Examples and Comparative Examples, but the present invention is not limited to the following Examples. The structural unit of the obtained synthetic resin, modified polyvinyl acetal resin was measured by dissolving the resin in DMSO-d6 (dimethyl sulfoxide) and using $^1$H-NMR (nuclear magnetic resonance spectrum). The structural unit is, for example, the content of a structural unit having an amine structure (mol%), the content of a structural unit having an imine structure (mol%), the content of a structural unit having an amide structure (mol%), the degree of acetalization (mol%), the amount of acetyl group (mol%), the amount of hydroxyl group (mol%) and the amount of (meth)acrylic ester group (mol%).

[Example 1]

(Preparation of polyvinyl butyral resin)

A reactor equipped with a stirrer was charged with 2700 mL of ion-exchanged water, and 300 g of polyvinyl alcohol having an average degree of polymerization of 250 and a degree of saponification of 99 mol%, followed by dissolution by heating with stirring to prepare a solution. Next, to the solution, 35% by weight hydrochloric acid as a catalyst was added such that the concentration of hydrochloric acid became 0.2% by weight, and after the temperature was adjusted to 15°C, 22 g of n-butyraldehyde (n-BA) was added while being stirred. Thereafter, when 148 g of n-butyraldehyde (n-BA) was added, a polyvinyl butyral resin was precipitated in the form of white particles. Fifteen minutes after the precipitation, 35% by weight hydrochloric acid was added such that the concentration of hydrochloric acid became 1.8% by weight, followed by heating to 50°C for aging at 50°C for 2 hours. Next, the solution was cooled and neutralized, and then the polyvinyl butyral resin was washed with water and dried.

The obtained polyvinyl butyral had an average degree of polymerization of 250, an amount of hydroxyl group of 28 mol%, an amount of acetyl group of 1 mol% and a degree of acetalization of 71 mol%.

(Preparation of container for cell culture)

By dissolving 1 g of the obtained polyvinyl butyral resin in 19 g of butanol, a solution of polyvinyl butyral resin was obtained. By discharging 150 μL of the obtained solution of polyvinyl butyral resin onto a φ22 mm cover glass (manufactured by Matsunami Glass Ind., Ltd., 22 round No. 1 was used after dust was removed with air duster) and spinning it at 2,000 rpm for 20 seconds using a spin coater, a smooth resin film was obtained. By placing the obtained resin film on a φ22 mm polystyrene dish together with the cover glass, a container for cell culture was obtained.

(Surface free energy)

The surface free energy of the resin film was measured using a contact angle meter (manufactured by Kyowa Interface Science, Inc., DMo-701). A contact angle of pure water was obtained by dropping 1 μL of pure water onto the resin film, and then photographing the droplet image after 30 seconds. In addition, a contact angle of diiodomethane was obtained by dropping 1 μL of diiodomethane onto the resin film, and then photographing the droplet image after 30 seconds. From the obtained contact angles, the dispersion component $\gamma^d$ and the dipole component $\gamma^p$ of the surface free energy were calculated using the Kaelble-Uy theoretical formula.

Using the obtained container for cell culture, tests were conducted under the following conditions.

(Method for cell culture test)

To the obtained container for cell culture, 1 mL of phosphate buffered saline was added, and the mixture was allowed to stand for 1 hour in an incubator at 37°C. After removing the phosphate buffered saline in the dish, $3\times10^4$ cells of human fresh hepatocytes derived from a chimeric mouse (PXB-cells manufactured by PhoenixBio Co., Ltd.) were seeded. Next, 1 mL of medium RM-101 (manufactured by Toyo Gosei Co., Ltd.) was added, followed by culture in an incubator at 37°C with a $CO_2$ concentration of 5%.

(Evaluation)

(1) Initial adhesion (fixation after seeding)

In the cell culture test, cells were detached using trypsin 24 hours after seeding of the cells. The number of cells was measured using an auto cell counter (Auto Cell Counter EVE, manufactured by NanoEnteck Inc.).

<Evaluation criteria for initial adhesion>

○○○: The number of cells is $2.5 \times 10^4$ cells or more
○○: The number of cells is $1.5 \times 10^4$ cells or more and less than $2.5 \times 10^4$ cells
○: The number of cells is $1.0 \times 10^4$ cells or more and less than $1.5 \times 10^4$ cells
×: The number of cells is less than $1.0 \times 10^4$ cells

(2) Cell proliferation

In the cell culture test, cells were detached using trypsin 3 days after seeding of the cells. The number of cells was measured using an auto cell counter (Auto Cell Counter EVE, manufactured by NanoEnteck Inc.).

<Evaluation criteria for cell proliferation>

○○○: The number of cells is $2.0 \times 10^5$ cells or more
○○: The number of cells is $1.0 \times 10^5$ cells or more and less than $2.0 \times 10^5$ cells
○: The number of cells is $5.0 \times 10^4$ cells or more and less than $1.0 \times 10^5$ cells
×: The number of cells is less than $5.0 \times 10^4$ cells

[Example 2]

The test was performed in the same manner as in Example 1 except that polyvinyl alcohol having an average degree of polymerization of 850 and a degree of saponification of 99 mol% was used.

[Example 3]

The test was performed in the same manner as in Example 1 except that polyvinyl alcohol having an average degree of polymerization of 1,700 and a degree of saponification of 99 mol% was used.

[Example 4]

The test was performed in the same manner as in Example 1 except that polyvinyl alcohol having an average degree of polymerization of 2,400 and a degree of saponification of 99 mol% was used, and that acetaldehyde was used instead of n-butyraldehyde (n-BA).

[Example 5]

The test was performed in the same manner as in Example 1 except that polyvinyl alcohol having an average degree of polymerization of 850, a degree of saponification of 98 mol%, and a degree of ethylene modification of 4 mol% was used.

[Example 6]

The test was performed in the same manner as in Example 1 except that polyvinyl alcohol having an average degree of polymerization of 250 and a degree of saponification of 99 mol%, and containing 2 mol% structural unit having an amino group represented by the formula (21) was used.

[Example 7]

The test was performed in the same manner as in Example 1 except that polyvinyl alcohol having an average degree of polymerization of 1,600 and a degree of saponification of 99 mol%, and containing 2 mol% structural unit having an amino group represented by the formula (21) was used.

[Example 8]

In 500 parts by weight tetrahydrofuran, 100 parts by weight polyvinyl acetal having a degree of polymerization of about 250 obtained in Example 1 and 1 part by weight N-vinylpyrrolidone were dissolved to prepare a graft copolymer resin solution. In the prepared resin solution, 0.05 parts by weight Irgacure184 (manufactured by BASF) was dissolved, and the resultant mixture was applied onto a PET film. The coated product was irradiated with light having a wavelength of 365 nm at an integrated light amount of 2000 mJ/$cm^2$ using a UV conveyor device "ECS301G1" manufactured by Eye Graphics Co., Ltd. at 25°C to prepare a composite resin solution. The prepared composite resin solution was vacuum-dried at 80°C for 3 hours to prepare a composite resin. The prepared composite resin was measured for weight average molecular weight in terms of polystyrene by GPC method using "2690 Separations Model" manufactured by Waters Corporation as a column. The weight average molecular weight was about 40,000. The prepared composite resin was adjusted to a 3% by weight butanol solution, and the test was conducted in the same manner as in Example 1.

[Example 9]

The test was performed in the same manner as in Example 8 except that 10 parts by weight N-vinylpyrrolidone was added to 100 parts by weight polyvinyl acetal. The weight average molecular weight of the obtained resin was about 60,000.

[Example 10]

The test was performed in the same manner as in Example 8 except that 30 parts by weight N-vinylpyrrolidone was added to 100 parts by weight polyvinyl acetal. The weight average molecular weight of the obtained resin was about 50,000.

[Example 11]

The test was performed in the same manner as in Example 8 except that 5 parts by weight tetrahydrofurfuryl acrylate was added to 100 parts by weight polyvinyl acetal. The weight average molecular weight of the obtained resin was about 60,000.

[Example 12]

The test was performed in the same manner as in Example 8 except that 5 parts by weight methoxyethyl acrylate was added to 100 parts by weight polyvinyl acetal. The weight average molecular weight of the obtained resin was about 70,000.

[Example 13]

The test was performed in the same manner as in Example 8 except that 5 parts by weight butyl methacrylate was added to 100 parts by weight polyvinyl acetal. The weight average molecular weight of the obtained resin was about 60,000.

[Example 14]

In 300 parts by weight tetrahydrofuran, 75 parts by weight N-isopropylacrylamide and 25 parts by weight butyl methacrylate were dissolved to prepare an acrylic monomer solution. In the prepared acrylic monomer solution, 2 parts by weight Irgacure184 (manufactured by BASF) was dissolved, and the resultant mixture was applied onto a PET film. The coated product was irradiated with light having a wavelength of 365 nm at an integrated light amount of 2,000 mJ/$cm^2$ using a UV conveyor device "ECS301G1" manufactured by Eye Graphics Co., Ltd. at 25°C to prepare an acrylic resin solution. The prepared acrylic resin solution was vacuum-dried at 80°C for 3 hours to prepare an acrylic resin. The prepared acrylic resin was adjusted to a 3% by weight butanol solution, and the test was conducted in the same manner as in Example 1. The weight average molecular weight of the obtained acrylic resin was about 100,000.

[Example 15]

An acrylic resin was obtained in the same manner as in Example 14 except that 90 parts by weight methoxyethyl acrylate and 10 parts by weight butyl methacrylate were used, instead of 75 parts by weight N-isopropylacrylamide and

25 parts by weight butyl methacrylate. The prepared acrylic resin was adjusted to a 3% by weight butanol solution, and the test was conducted in the same manner as in Example 1. The weight average molecular weight of the obtained acrylic resin was about 80,000.

[Example 16]

An acrylic resin was obtained in the same manner as in Example 14 except that 75 parts by weight methoxyethyl acrylate and 25 parts by weight butyl methacrylate were used, instead of 75 parts by weight N-isopropylacrylamide and 25 parts by weight butyl methacrylate. The prepared acrylic resin was adjusted to a 3% by weight butanol solution, and the test was conducted in the same manner as in Example 1. The weight average molecular weight of the obtained resin was about 90,000.

[Example 17]

An acrylic resin was obtained in the same manner as in Example 14 except that 2 parts by weight butyl methacrylate and 98 parts by weight ethyl acrylate were used, instead of 75 parts by weight N-isopropylacrylamide and 25 parts by weight butyl methacrylate. The prepared acrylic resin was adjusted to a 3% by weight butanol solution, and the test was conducted in the same manner as in Example 1. The weight average molecular weight of the obtained acrylic resin was about 80,000.

[Comparative Example 1]

The test was performed in the same manner as in Example 1 using only a polystyrene dish without using the scaffold material.

[Comparative Example 2]

The test was performed in the same manner as in Example 1 except that the amount of the second addition of n-butyraldehyde (n-BA) was changed from 148 g to 89 g.

[Comparative Example 3]

The test was performed in the same manner as in Example 1 except that polyvinyl alcohol having an average degree of polymerization of 1,000 and a degree of saponification of 98 mol% was used as the synthetic resin.

[Comparative Example 4]

A polyacrylamide resin was obtained by mixing 100 parts by weight N-isopropylacrylamide, 75 parts by weight ethyl acetate and 0.5 parts by weight azobisisobutyronitrile, followed by polymerization at 65°C for 8 hours under a nitrogen atmosphere. The prepared resin was measured for weight average molecular weight in terms of polystyrene by GPC method using "2690 Separations Model" manufactured by Waters Corporation as a column. The weight average molecular weight was about 90,000 (the degree of polymerization was about 800). Other operations in the test were performed in the same manner as in Example 1.

[Comparative Example 5]

The test was performed in the same manner as in Comparative Example 4 except that 100 parts by weight ethyl acrylate was used instead of 100 parts by weight N-isopropylacrylamide.

[Comparative Example 6]

The test was performed in the same manner as in Comparative Example 4 except that 100 parts by weight butyl methacrylate was used instead of 100 parts by weight N-isopropylacrylamide. The weight average molecular weight of the obtained resin was about 90,000.

[Comparative Example 7]

The test was performed in the same manner as in Example 8 except that 70 parts by weight N-vinylpyrrolidone

was added to 30 parts by weight polyvinyl acetal. The weight average molecular weight of the obtained resin was about 90,000.

The obtained results are summarized in Tables 1 to 4.

[Table 1]

| | | | Example 1 | Example 2 | Example 3 | Example 4 | Example 5 |
|---|---|---|---|---|---|---|---|
| Synthetic resin | Polyvinyl acetal resin | Degree of acetalization (mol%) | 71 | 68 | 65 | 66 | 64 |
| | | Amount of acetyl group (mol%) | 1 | 1 | 1 | 1 | 2 |
| | | Amount of hydroxyl group (mol%) | 28 | 31 | 34 | 33 | 30 |
| | | Content of structural unit having amine structure (1) (mol%) | | | | | |
| | | Content of structural unit having imine structure (2) (mol%) | | | | | |
| | | Content of structural unit having amide structure (3) (mol%) | | | | | |
| | | Total content of structural unit having amine structure, structural unit having imine group and structural unit having amide structure ((1) + (2) + (3)) (mol%) | | | | | |
| | | Average Degree of polymerization | 250 | 850 | 1700 | 2400 | 850 |
| | Surface free energy | $\gamma^d$ (mJ/m$^2$) | 32.5 | 32.6 | 33.5 | 31.3 | 34.2 |
| | | $\gamma^p$ (mJ/m$^2$) | 3.5 | 3.7 | 3.5 | 4.6 | 3.3 |
| Evaluation for culture | Initial adhesion | | ○ | ○ | ○ | ○ | ○ |
| | Cell proliferation | | ○ | ○ | ○ | ○ | ○ |

[Table 2]

| | | | Example 6 | Example 7 | Example 8 | Example 9 | Example 10 |
|---|---|---|---|---|---|---|---|
| Synthetic resin | Polyvinyl acetal resin | Degree of acetalization (mol%) | 77 | 76 | 70 | 65 | 54 |
| | | Amount of acetyl group (mol%) | 1 | 1 | 1 | 1 | 1 |
| | | Amount of hydroxyl group (mol%) | 20 | 21 | 28 | 25 | 21 |
| | | Content of structural unit having amine structure (1) (mol%) | 0.3 | 0.3 | - | - | - |
| | | Content of structural unit having imine structure (2) (mol%) | 1.7 | 1.7 | - | - | - |
| | | Content of structural unit having amide structure (3) (mol%) | - | - | 1 | 9 | 24 |
| | | Total content of structural unit having amine structure, structural unit having imine group and structural unit having amide structure ((1) + (2) + (3)) (mol%) | 2.0 | 2.0 | 1 | 9 | 24 |
| | | Average Degree of polymerization | 250 | 1600 | 250 | 250 | 250 |
| | Surface free energy | $\gamma^d$ (mJ/m$^2$) | 34.2 | 34.8 | 33.0 | 35.7 | 36.2 |
| | | $\gamma^p$ (mJ/m$^2$) | 3.3 | 3.6 | 3.2 | 2.6 | 2.2 |
| Evaluation for culture | Initial adhesion | | ○○ | ○○ | ○○○ | ○○○ | ○○ |
| | Cell proliferation | | ○○ | ○○ | ○○○ | ○○○ | ○○ |

[Table 3]

| | | | Example 11 | Example 12 | Example 13 | Example 14 | Example 15 | Example 16 | Example 17 |
|---|---|---|---|---|---|---|---|---|---|
| Synthetic resin | Polyvinyl acetal resin | Degree of acetalization (mol%) | 68 | 68 | 68 | - | - | - | - |
| | | Amount of acetyl group (mol%) | 1 | 1 | 1 | - | - | - | - |
| | | Amount of hydroxyl group (mol%) | 27 | 27 | 27 | - | - | - | - |
| | | Content of structural unit having amine structure (1) (mol%) | | | | | | | |
| | | Content of structural unit having imine structure (2) (mol%) | | | | | | | |
| | | Content of structural unit having amide structure (3) (mol%) | | | | | | | |
| | | Content of structural unit derived from tetrahydrofurfuryl acrylate (mol%) | 4 | - | - | - | - | - | - |
| | | Content of structural unit derived from methoxyethyl acrylate (mol%) | - | 4 | - | - | - | - | - |
| | | Content of structural unit derived from butyl methacrylate (mol%) | - | - | 4 | - | - | - | - |
| | | Total content of structural unit having amine structure, structural unit having imine group and structural unit having amide structure ((1) + (2) + (3)) (mol%) | - | - | - | - | - | - | - |
| | | Average degree of polymerization | 250 | 250 | 250 | - | - | - | - |
| | Poly(meth) acrylic ester | Content of structural unit having amide structure (mol%) | - | - | - | 79 | - | - | - |
| | | Content of structural unit derived from butyl methacrylate (mol%) | - | - | - | 21 | 9 | 23 | 1 |
| | | Content of structural unit derived from methoxyethyl acrylate (mol%) | - | - | - | - | 91 | 77 | - |
| | | Content of structural unit derived from ethyl acrylate (mol%) | - | - | - | - | - | - | 99 |
| | | Average Degree of polymerization | - | - | - | 250 | 250 | 250 | 250 |

(continued)

| | | | Example 11 | Example 12 | Example 13 | Example 14 | Example 15 | Example 16 | Example 17 |
|---|---|---|---|---|---|---|---|---|---|
| | Surface free energy | $\gamma^d$ (mJ/m$^2$) | 35.1 | 34 | 34.4 | 24.8 | 38.9 | 42.9 | 42.7 |
| | | $\gamma^p$ (MJ/m$^2$) | 2.9 | 4.2 | 2.4 | 9.2 | 17.9 | 8.6 | 5.8 |
| Evaluation for culture | Initial adhesion | | ○○ | ○○ | ○○ | ○ | ○ | ○ | ○ |
| | Cell proliferation | | ○○ | ○○ | ○○ | ○ | ○ | ○ | ○ |

[Table 4]

| | | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| | Polyvinyl acetal resin | Degree of acetalization (mol%) | - | 40 | 0 | - | - | - | 21 |
| | | Amount of acetyl group (mol%) | - | 3 | 2 | - | - | - | 0 |
| | | Amount of hydroxyl group (mol%) | - | 57 | 98 | - | - | - | 8 |
| | | Content of structural unit having amine structure (1) (mol%) | - | - | - | - | - | - | - |
| | | Content of structural unit having imine structure (2) (mol%) | - | - | - | - | - | - | - |
| | | Content of structural unit having amide structure (3) (mol%) | - | - | - | - | - | - | 71 |
| | | Content of structural unit derived from tetrahydrofurfuryl acrylate (mol%) | - | - | - | - | - | - | - |
| | | Content of structural unit derived from methoxyethyl acrylate (mol%) | - | - | - | - | - | - | - |
| | | Content of structural unit derived from butyl methacrylate (mol%) | - | - | - | - | - | - | - |

(continued)

| | | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| Synthetic resin | | Total content of structural unit having amine structure, structural unit having imine group and structural unit having amide structure ((1) + (2) + (3)) (mol%) | - | - | - | - | - | - | 71 |
| | | Average degree of polymerization | - | 250 | 1000 | - | - | - | 250 |
| | Poly (meth) acrylic ester | Content of structural unit having amide structure (mol%) | - | - | - | 100 | - | - | - |
| | | Content of structural unit derived from butyl methacrylate (mol%) | - | - | - | - | - | 100 | - |
| | | Content of structural unit derived from methoxyethyl acrylate (mol%) | - | - | - | - | - | - | - |
| | | Content of structural unit derived from ethyl acrylate (mol%) | - | - | - | - | 100 | - | - |
| | | Average degree of polymerization | - | - | - | 800 | 600 | 550 | 250 |
| | Surface free energy | $\gamma^d$ (mJ/m$^2$) | 45.8 | 28.9 | 26.7 | 24.0 | 23.1 | 45.7 | 24.3 |
| | | $\gamma^p$ (mJ/m$^2$) | 5.8 | 26.8 | 34.9 | 19.6 | 6.0 | 1.0 | 26.8 |
| Evaluation for culture | Initial adhesion | | × | × | × | × | × | × | × |
| | Cell proliferation | | × | × | × | × | × | × | × |

## Claims

1. A scaffolding material for cell culture,

   the scaffolding material for cell culture
   having a dispersion component $\gamma^d$ of surface free energy of 28.0 mJ/m$^2$ or more and 38.0 mJ/m$^2$ or less,
   having a dipole component $\gamma^p$ of surface free energy of 1.0 mJ/m$^2$ or more and 10.0 mJ/m$^2$ or less,
   comprising a synthetic resin, and
   being used for culturing a progenitor cell or a differentiated cell,
   the synthetic resin having at least one skeleton of a polyvinyl acetal skeleton and a poly(meth)acrylic ester skeleton.

2. The scaffolding material for cell culture according to claim 1, wherein the synthetic resin is a polyvinyl acetal resin.

3. A scaffolding material for cell culture,

   the scaffolding material for cell culture comprising a synthetic resin, and being used for culturing a progenitor cell or a differentiated cell,
   the synthetic resin including a polyvinyl acetal resin,
   a degree of acetalization of the polyvinyl acetal resin being higher than 60 mol%, and
   the polyvinyl acetal resin being a polyvinyl butyral resin.

4. The scaffolding material for cell culture according to claim 2 or 3, wherein the polyvinyl acetal resin has at least one structural unit selected from the group consisting of a structural unit having an amine structure, a structural unit having an imine structure and a structural unit having an amide structure.

5. The scaffolding material for cell culture according to claim 4, wherein the polyvinyl acetal resin has a total content of the structural unit having an amine structure, the structural unit having an imine structure and the structural unit having an amide structure of 0.1 mol% or more and 30 mol% or less.

6. A container for cell culture, comprising the scaffolding material for cell culture according to any one of claims 1 to 5 on at least a part of a cell culture region.

7. A fiber for cell culture, comprising the scaffolding material for cell culture according to any one of claims 1 to 5.

8. A method for culturing a cell, comprising using the scaffolding material for cell culture according to any one of claims 1 to 5.

9. The method for culturing a cell according to claim 8, comprising a step of seeding a cell mass on the scaffolding material for cell culture.

[FIG. 1.]

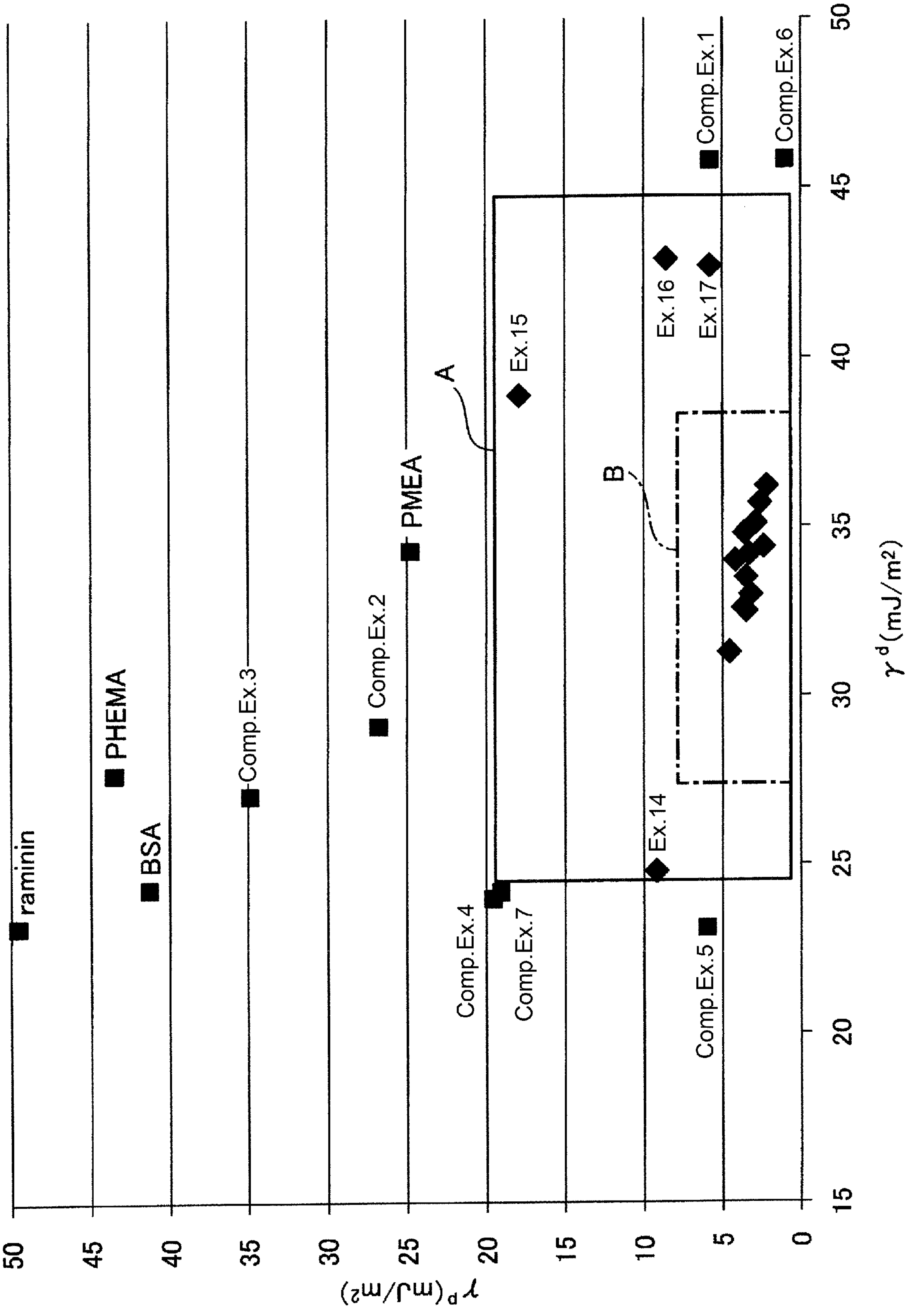
raminin
PHEMA
BSA
Comp.Ex.3
Comp.Ex.2
PMEA
A
Ex.15
B
Ex.16
Ex.17
Comp.Ex.1
Comp.Ex.6
Ex.14
Comp.Ex.4
Comp.Ex.7
Comp.Ex.5
$\gamma^d$(mJ/m²)
$\gamma^p$(mJ/m²)
15
20
25
30
35
40
45
50
0
5
10
15
20
25
30
35
40
45
50

[FIG. 2.]

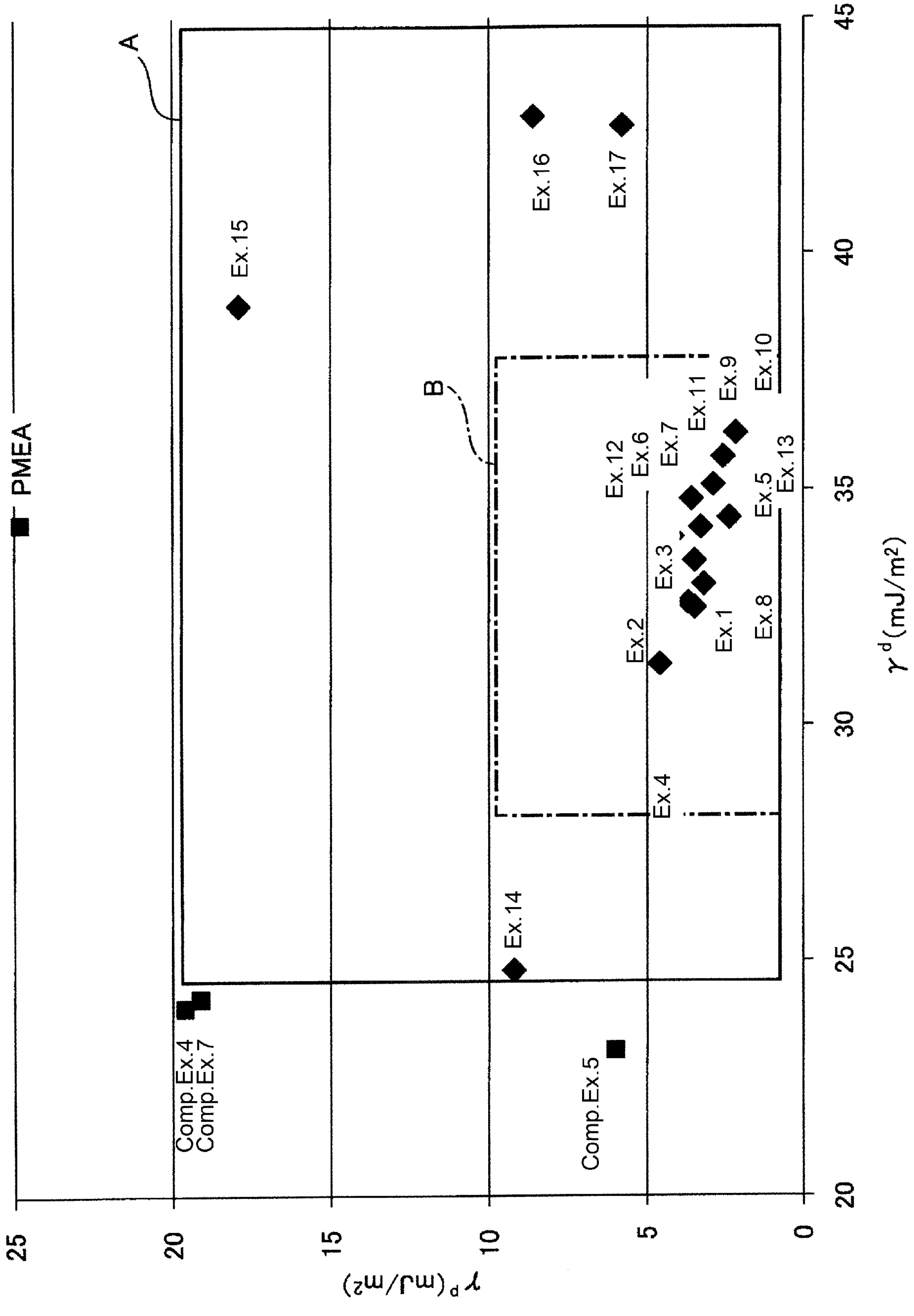
PMEA
A
B
Ex.1
Ex.2
Ex.3
Ex.4
Ex.5
Ex.6
Ex.7
Ex.8
Ex.9
Ex.10
Ex.11
Ex.12
Ex.13
Ex.14
Ex.15
Ex.16
Ex.17
Comp.Ex.4
Comp.Ex.7
Comp.Ex.5
γ^d (mJ/m²)
γ^p (mJ/m²)
20
25
30
35
40
45
0
5
10
15
20
25

[FIG. 3.]

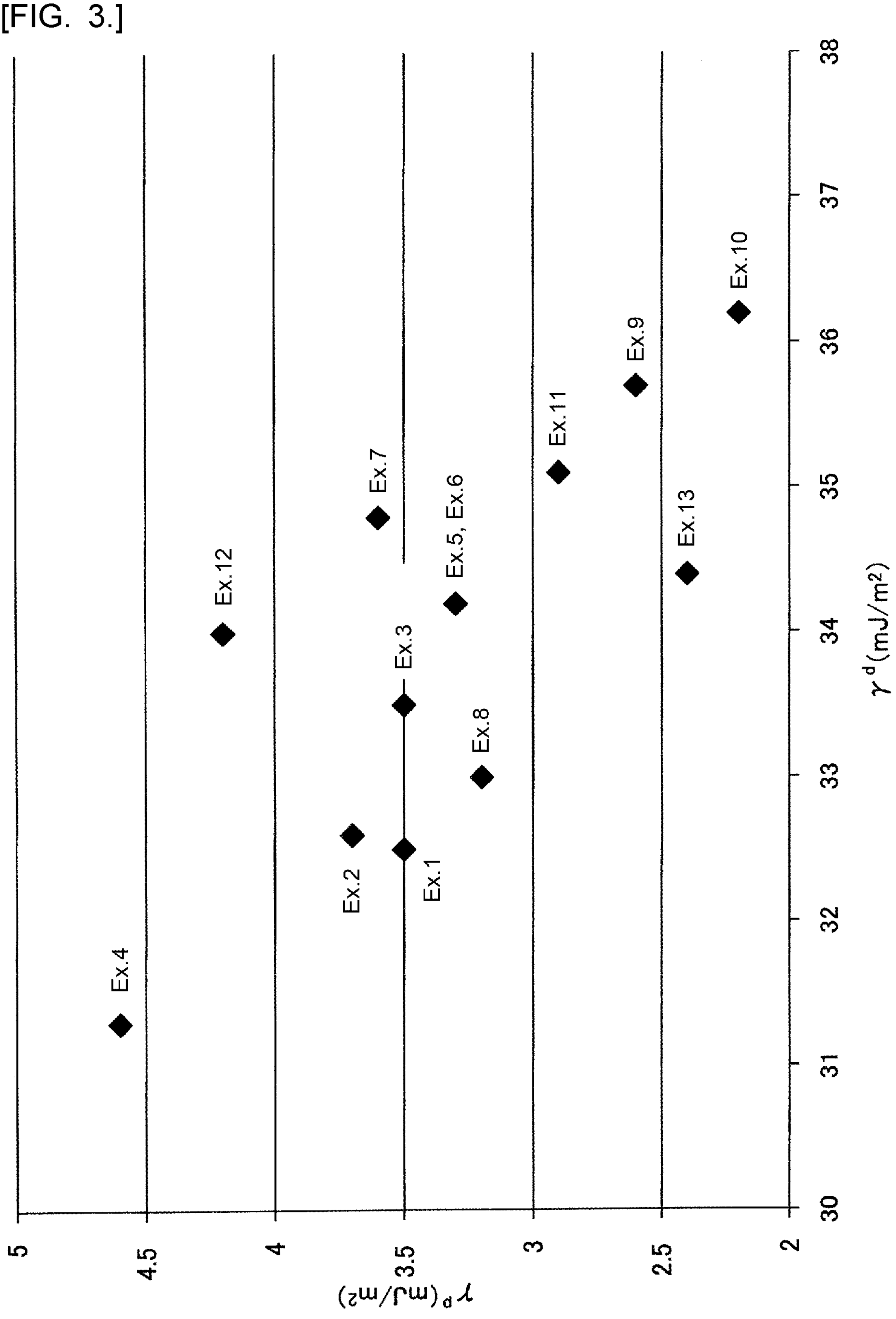

γ^d(mJ/m²)
γ^p(mJ/m²)
30
31
32
33
34
35
36
37
38
2
2.5
3
3.5
4
4.5
5
Ex.1
Ex.2
Ex.3
Ex.4
Ex.5, Ex.6
Ex.7
Ex.8
Ex.9
Ex.10
Ex.11
Ex.12
Ex.13

## INTERNATIONAL SEARCH REPORT

International application No. PCT/JP2020/014013

A. CLASSIFICATION OF SUBJECT MATTER
Int.Cl. C12M1/00(2006.01)i. C12M3/00(2006.01)i. C12N5/00(2006.01)i. C12N5/07(2010.01)i
FI: C12M3/00A. C12M1/00A. C12N5/00. C12N5/07
According to International Patent Classification (IPC) or to both national classification and IPC

B. FIELDS SEARCHED
Minimum documentation searched (classification system followed by classification symbols)
Int.Cl. C12M1/00. C12M3/00. C12N5/00. C12N5/07

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDreamIII);
CAplus/MEDLINE/EMBASE/BIOSIS/WPIDS(STN); WPIDS/WPIX(STN)

C. DOCUMENTS CONSIDERED TO BE RELEVANT

| Category* | Citation of document, with indication, where appropriate, of the relevant passages | Relevant to claim No. |
|---|---|---|
| X<br>Y | US 4537790 A (INSTITUT PASTEUR) 27.08.1985 (1985-08-27), claims | 1-3, 6-9<br>3-9 |
| X<br>Y | JP 02-227070 A (ASAHI OPTICAL CO., LTD.) 10.09.1990 (1990-09-10), claims, example 3, page 3, upper left column, lines 11-18 | 1-3, 7<br>3-9 |
| Y | JP 05-076364 A (KANEGAFUCHI CHEM IND CO., LTD.) 30.03.1993 (1993-03-30), paragraph [0015] | 3-9 |
| Y | JP 2000-328356 A (NITIVY CO., LTD.) 28.11.2000 (2000-11-28), claims, examples | 4-9 |
| Y | JP 10-158322 A (HYMO CORPORATION) 16.06.1998 (1998-06-16), claims, examples | 4-9 |
| Y | JP 10-287711 A (UNITIKA CHEM CO., LTD.) 27.10.1998 (1998-10-27), claims, examples | 4-9 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
08.06.2020

Date of mailing of the international search report
16.06.2020

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No. PCT/JP2020/014013

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-237158 A (AION KK) 09.10.2008 (2008-10-09), entire text | 1-9 |
| A | JP 2004-129572 A (BIO ENERGY KK) 30.04.2004 (2004-04-30), entire text | 1-9 |
| P, X | WO 2019/131978 A1 (SEKISUI CHEMICAL CO., LTD.) 04.07.2019 (2019-07-04), entire text | 1-9 |
| P, X | WO 2019/131981 A1 (SEKISUI CHEMICAL CO., LTD.) 04.07.2019 (2019-07-04), entire text | 1-9 |

Form PCT/ISA/210 (second sheet) (January 2015)

**INTERNATIONAL SEARCH REPORT**
Information on patent family members

International application No.
PCT/JP2020/014013

| | | |
|---|---|---|
| US 4537790 A | 27.08.1985 | BE 869459 A |
| JP 02-227070 A | 10.09.1990 | US 5085781 A<br>claims, example 3, pillar 4 line 68 to pillar 5, line 13 |
| JP 05-076364 A | 30.03.1993 | (Family: none) |
| JP 2000-328356 A | 28.11.2000 | (Family: none) |
| JP 10-158322 A | 16.06.1998 | (Family: none) |
| JP 10-287711 A | 27.10.1998 | (Family: none) |
| JP 2008-237158 A | 09.10.2008 | (Family: none) |
| JP 2004-129572 A | 30.04.2004 | (Family: none) |
| WO 2019/131978 A1 | 04.07.2019 | TW 201930587 A |
| WO 2019/131981 A1 | 04.07.2019 | TW 201930587 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006314285 A
- JP 2010158180 A
- JP 2017023008 A